# FASCICULE DE BREVET EUROPEEN

(11) **EP 1 056 407 B1**
(45) Date de publication et mention de la délivrance du brevet: **07.07.2004**
(21) Numéro de dépôt: 99958340.4
(22) Date de dépôt: 16.12.1999
(51) Int. Cl.: A61B 17/64

(54) **DISPOSITIF ORTHOPEDIQUE MONOLATERAL DE FIXATION EXTERNE**
ORTHOPÄDISCHER MONOLATERALER AUSSENFIXATEUR
MONOLATERAL ORTHOPAEDIC DEVICE WITH EXTERNAL FIXING

(30) Priorité: 29.12.1998 FR 9816524
(43) Date de publication de la demande: 06.12.2000
(73) Titulaire: Etat Français représenté par le Délégué Général pour l' Armement, 00457 Armées (FR); Di Shino, Michel, 92140 Clamart (FR); Steenman, Christian, 78000 Versailles (FR); Dalzotto, Georges, 92121 Issy-les-Moulineaux (FR); Rigal, Sylvain, 92140 Clamart (FR)
(72) Inventeur: DI SHINO, Michel, F-92140 Clamart (FR); STEENMAN, Christian, F-78000 Versailles (FR); DALZOTTO, Georges, F-92121 Issy-les-Moulineaux (FR); RIGAL, Sylvain, F-92140 Clamart (FR); EVRARD, Patrick, F-45000 Orléans (FR)
(86) Numéro de dépôt international: PCT/FR1999/003158
(87) Numéro de publication internationale: WO 2000/038585

(56) Documents cités:
- WO-A-97/35527
- DE-A- 3 539 616
- FR-A- 2 536 984
- US-A- 4 620 533
- US-A- 5 624 440

## Description

Le secteur technique de la présente invention est celui des dispositifs orthopédiques à usage externe destinés à l'immobilisation de membres fracturés et à la réduction des fractures. Ces types de dispositifs sont utilisés dans le domaine de l'ostéosynthèse par fixateur externe.

Le traitement des fractures ouvertes, souillées, à large ouverture, anciennes ou causées par des projectiles à grande énergie cinétique, fait appel à la fixation externe.

La fixation d'une fracture, en particulier à foyer ouvert doit être stable. En effet, des contraintes de compression traction, de torsion et de flexion, situées au niveau du foyer de la fracture sont transmises au fixateur externe par l'intermédiaire de la liaison os / fiche.

La rigidité du fixateur intervient de ce fait de manière prépondérante dans la consolidation des fragments osseux d'une fracture.

Les fixateurs externes existant actuellement sur le marché sont nombreux et de différents types : unilatéraux, en cadre ou encore circulaires.

L'expérience montre que la majorité des fixateurs posés en urgence dans le cas notamment de la chirurgie de guerre ou de catastrophe nécessite une reprise, le plus souvent en raison de défauts de réduction ; la mise en place d'emblée idéale est rare.

La reprise du montage initial ne laisse que deux possibilités, soit :
- enlever les fiches, refaire la réduction et remettre les fiches à travers une nouvelle barre de liaison, on perd ainsi l'intérêt de la fixation initiale ;
- laisser les fiches, la réduction nécessitant alors de modifier le montage avec deux barres courtes supérieure et inférieure réunies par un moyen d'union, le montage perd alors sa rigidité initiale.

On connaît des fixateurs externes, composés de tubes rigides ou moyens équivalents dans lesquels des fiches sont engagées et bloquées par des vis, décrits par exemple dans les brevets FR 2 442 044, FR 2 551 650 et FR 2 553 994.

En particulier, le brevet FR 2 457 676 décrit un fixateur de type unilatéral permettant la réduction d'une fracture. Il comprend un tube principal supportant deux segments de tubes assemblés sur le premier à l'aide de rotules sphériques. Le coulissement des deux segments par rapport au tube principal est obtenu par des colliers, chaque tube présentant dans deux plans perpendiculaires entre eux des orifices diamétralement opposés régulièrement espacés et filetés, destinés à recevoir les fiches et les vis de blocage.

Ces fixateurs, portés par des patients en mouvement, présentent de nombreux inconvénients :
- sa mise en place dans les cas d'urgence nécessite une réduction et une contention provisoire du foyer osseux, ou du moins l'alignement correct du membre en cas de perte de substance osseuse, avant l'implantation des fiches ; cette contrainte d'emploi entraîne de nombreuses implantations initiales imparfaites,
- l'espacement des fiches est strictement défini par les trous des tubes et ces fiches sont situées dans un plan unique et sont parallèles entres-elles ; ce principe ne permet aucune modularité de l'orientation ou de l'espacement des fiches implantées dans l'os,
- autour d'un appareil comprenant cinq éléments constitutifs, à savoir : tube principal, segment de tube, rotule, collier et fiche, s'est développé un véritable arsenal de pièces de différents diamètres ; le nombre élevé de pièces rend les montages complexes et est à l'origine de perte de temps, lors d'une utilisation par un chirurgien peu accoutumé à son emploi,
   les différents éléments de base ne sont pas radiotransparents et sont lourds, car réalisés en métal ; ce dernier point est un inconvénient pour le patient, mais également lors du transport des boîtes d'instrumentation dans le cas notamment des antennes mobiles chirurgicales.

Le brevet US 4 483 334 décrit un appareil de fixation externe des fractures comprenant des fiches vissées dans l'os de part et d'autre de la fracture, une paire de tiges reliées à ces fiches de part et d'autre de la fracture et un pont reliant les tiges entre elles. Les mécanismes de liaison reliant chacune des fiches au tiges sont constitués par l'association d'une prise de fiche monobloc en forme de U montée par clipsage sur les fiches et d'un collier adapté au diamètre des tiges. Les fiches, la tige, les prises de fiche et les colliers sont solidarisés par une vis unique assurant un blocage omnidirectionnel.

Le certificat d'utilité DE 91 03 480 décrit un dispositif d'immobilisation d'un doigt ou d'une main en cas de fracture comportant des pointes vissées de part et d'autre de la fracture, maintenues entre elles par une tige de raccordement. Le mécanisme de liaison entre les pointes et la tige de raccordement est constitué par un collier en forme de U, d'une douille, d'une vis dans laquelle est pratiqué un perçage pour le passage de la pointe, et d'un écrou. Le serrage de l'écrou assure le blocage de la pointe.

Ces deux dispositifs ne permettent la fixation que d'une fiche par mécanisme de liaison.

Le brevet DE 295 12 917 décrit un dispositif orthopédique comprenant notamment une prise de fiches permettant de maintenir simultanément au moins deux fiches en parallèle.

Cette prise est composée de deux pièces identiques formant un étau avec, sur les faces en regard, des empreintes permettant de saisir et maintenir les fiches.

Cette prise présente l'inconvénient de ne permettre le maintien qu'un seul diamètre de fiche. A chaque diamètre de fiche correspond un type de prise. Il faut donc posséder des prises de différentes configurations.

Le brevet DE 3 539 616 décrit un fixateur muni en particulier d'une prise de fiche ayant la forme d'un étau pouvant recevoir simultanément quatre fiches disposées en parallèles, grâce à des empreintes ménagées sur les deux faces en regard de la prise. On retrouve ici des inconvénients semblables à ceux mentionnés ci-dessus pour le brevet précédent.

Il existe d'autres dispositifs orthopédiques plus légers que les précédents, en matériaux radiotransparent et amagnétiques, tel le fixateur décrit par exemple par le brevet FR 2 688 685.

Ce fixateur comporte une tige rigide pleine présentant des orifices transversaux pour le passage des fiches vissées dans l'os, celles-ci étant solidarisées avec la tige par des inserts de fixation ou des colliers de serrage. L'espacement des orifices et donc l'espacement des fiches sont strictement définis et invariables.

Un objectif de la présente invention est de procurer un dispositif orthopédique de fixation externe compatible avec les fixateurs existants, notamment ceux utilisés dans les pays membre de l'Organisation du Traité de l'Atlantique Nord (OTAN) et permettant de réduire avec précision les fractures grâce à des réglages précis à plusieurs degrés de liberté.

Un autre objectif de la présente invention est de procurer un dispositif orthopédique qui permet de simplifier et de faciliter la pose du matériel par un chirurgien non-spécialiste, en offrant notamment la possibilité de reprise de défauts d'axe sans modification des fiches en place, ou encore la réalisation de prises épiphysaires.

Le but de la présente invention est de proposer un fixateur externe possédant une bonne rigidité et permettant à la fois :
- une simplification de la reprise des défauts d'axe, sans modification des fiches en place,
- une compatibilité avec les fiches des différents fixateurs utilisables, notamment avec ceux utilisés dans les pays de l'OTAN,
- une grande simplicité d'utilisation pour le chirurgien non-spécialiste, grâce à un nombre très faible de pièces constitutives,
- une réduction des fractures avec le fixateur en place,
- un montage monoplan ou au moins unilatéral permettant la confection de lambeaux musculaires de couverture aux membres concernés,
- une réalisation très simple de prises épiphysaires,
- une utilisation de fiches autoforeuses et autotaraudeuses de différents diamètres adaptés aux types d'interventions,
- une totale radiotransparence facilitant le suivi de la consolidation de l'os par radiographie ou imagerie par résonance magnétique,
- une obtention d'un certain confort pour le patient, eu égard à la légèreté des implants.

Pour ce faire le dispositif orthopédique monolatéral de fixation externe pour l'immobilisation d'un os fracturé comporte, de façon connue, un support rigide disposé parallèlement à l'os, au moins deux fiches vissées dans l'os et solidarisées avec le support rigide par un mécanisme de liaison comprenant une prise de fiches constituée de deux pièces identiques formant un étau, dont les deux faces en regard présentent des empreintes.

Ce dispositif est caractérisé en ce que les empreintes sont aptes à recevoir et à maintenir des fiches ayant des diamètres de 3 à 6 mm.

De préférence, les empreintes du dispositif ont des directions différentes, les empreintes dans l'une des directions permettent le maintien des fiches de 3 ou 4 mm de diamètre parallèles entre elles, les empreintes dans l'autre direction, perpendiculaire à la précédente, permettent le maintien de fiches de 5 ou 6 mm de diamètre parallèles entre elles.

De préférence, le mécanisme de liaison comporte un collier pouvant glisser autour du support et une vis unique amovible assurant le blocage omnidirectionnel à la fois de chaque mécanisme de liaison et de chaque fiche par rapport respectivement au support et à sa prise de fiches correspondante.

La prise de fiche peut comporter un mécanisme de détrompage. Les deux pièces identiques formant étau comportent chacune un ergot et une languette se logeant respectivement dans un orifice et une encoche de l'autre pièce identique. Ce mécanisme guide le positionnement de l'une des deux pièces par rapport à l'autre sans hésitation.

De préférence, le dispositif comporte des moyens d'assemblage des prises de fiches avec les colliers. Ces moyens peuvent comporter deux surfaces crénelées réalisées respectivement sur chaque face en contact des prises de fiches et de chaque collier, pour assurer un indexage précis, une cohésion des pièces entre elles et une totale immobilisation des fiches par rapport au support rigide.

De préférence, les colliers réalisés en matériau souple sont montés par clipsage sur le support rigide et le support transversal. Ils peuvent comporter une entretoise pour maintenir un écart constant entre les deux extrémités du collier et limiter le couple de serrage de la vis.

De préférence, le support rigide est constitué d'un tube cylindrique réalisé en carbone.

De préférence, la totalité du dispositif, excepté les fiches, est réalisée en matériau synthétique radiotransparent et amagnétique.

Selon une variante de réalisation, le dispositif peut comporter un support transversal, solidarisé au support rigide par un collier, pour maintenir une épiphyse de l'os fracturé à l'aide d'au moins une fiche vissée dans l'épiphyse et d'un mécanisme de liaison monté sur le support transversal.

Les différents types de pièces constitutives du dispositif peuvent comporter des couleurs distinctes pour faciliter leur identification lors du montage du dispositif sur le patient.

Ce dispositif présente l'avantage de comporter un nombre de pièces très faible, permettant un gain de temps important et une grande facilité de montage indispensables lors d'interventions réalisées dans l'urgence par un chirurgien non expérimenté.

Un autre avantage réside dans les liaisons support / collier et collier / prise autorisant un degré de liberté des fiches dans toutes les directions, ce qui permet d'adapter le dispositif lors de la reprise de défauts d'axe, sans modification des fiches en place.

De part sa réalisation en matériau synthétique très léger du type carbone, il facilite la lecture des radiographies de consolidation ainsi que le transport du dispositif par le chirurgien ou le patient.

Un autre avantage réside aussi dans la totale compatibilité des deux types de prises de fiches avec l'ensemble des fiches utilisées pour ce genre d'interventions.

D'autres caractéristiques et avantages de l'invention apparaîtront dans la description détaillée, non limitative, ci-dessous.

La description suivante se réfère aux dessins annexés qui représentent, sans aucun caractère limitatif, un exemple de réalisation d'un dispositif orthopédique de fixation externe selon la présente invention.

La figure 1 représente une vue en perspective d'un dispositif de fixation externe dans sa condition d'utilisation, par exemple pour l'immobilisation d'une fracture d'un os long du type fémur.

La figure 2 est une vue du dispositif selon la figure 1, montrant le montage d'une prise épiphysaire.

La figure 3 représente une vue partielle en perspective d'un montage des moyens de solidarisation d'une fiche sur un support rigide du dispositif, avec une prise de fiches monobloc.

Les figures 3A et 3B représentent le montage de la figure 3 en coupes transversales.

La figure 4 représente une prise de fiches monobloc en élévation.

La figure 5 représente une vue en élévation d'une prise de fiches en forme d'étau selon l'invention, montrant ses deux pièces identiques dissociées avec leurs faces externe et interne.

La figure 5A représente une vue en plan de la face interne de l'une des pièces identiques de la figure 5.

La figure 5B représente une vue en coupe selon AA de la figure 5B.

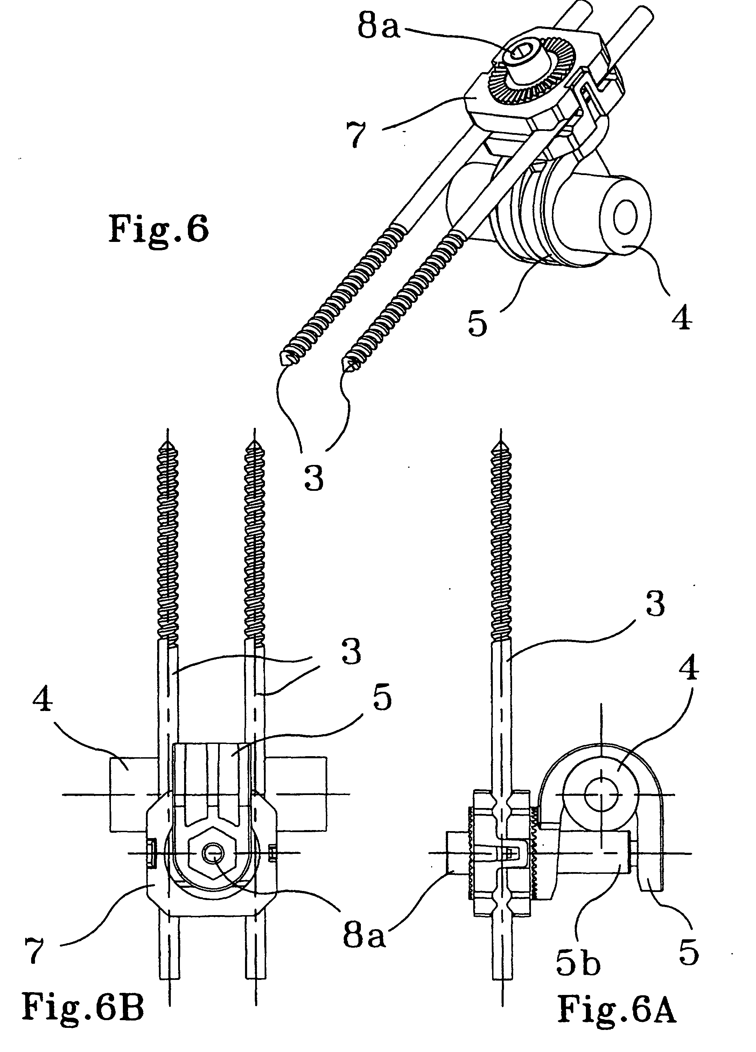

La figure 6 représente une vue selon la figure 3, d'un montage de deux fiches sur un support rigide, à l'aide d'une prise de fiches en forme d'étau.

Les figures 6A et 6B représentent le montage de la figure 6 en coupes transversales.

La figure 7 représente deux vues en élévation d'un collier selon l'invention.

La figure 8 représente une vue partielle en élévation du montage épiphysaire montré à la figure 2.

La figure 8A représente le montage selon la figure 8 en vue de dessus.

La figure 8B représente une vue en coupe partielle selon AA de la figure 8A.

En référence aux figures annexées on voit un dispositif orthopédique monolatéral de fixation externe utilisé pour l'immobilisation temporairement d'un os fracturé 2.

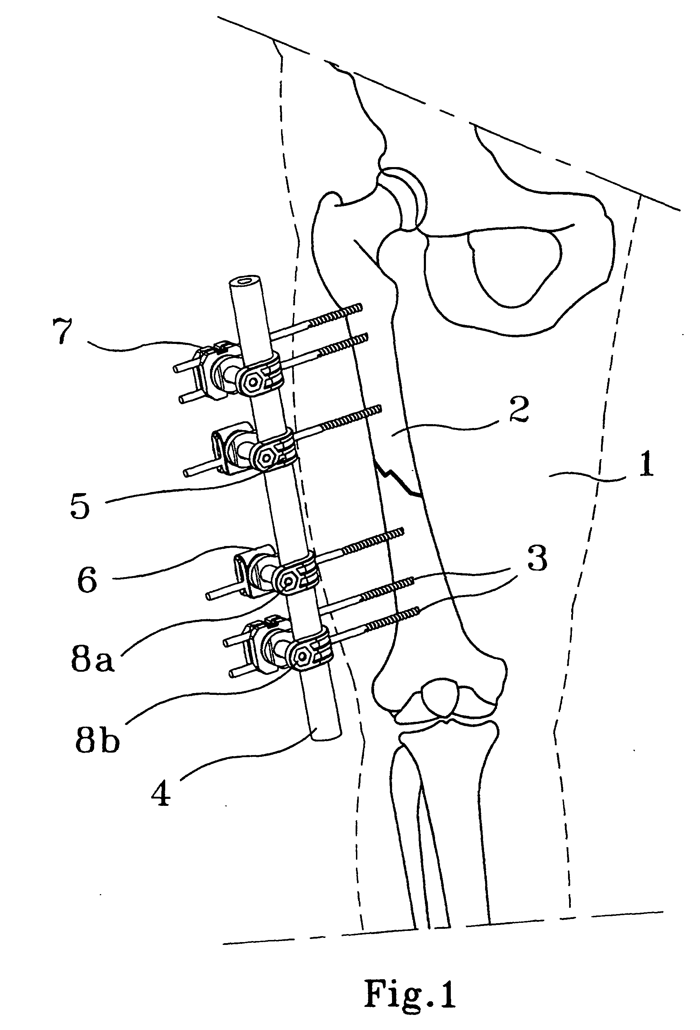

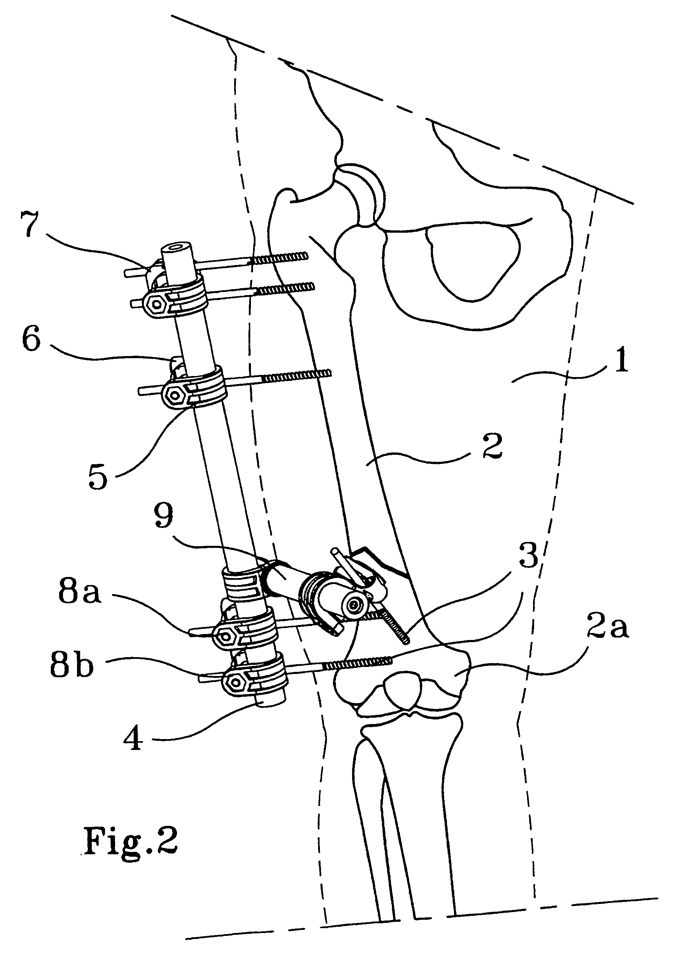

A titre d'exemple, les figures 1 et 2 montrent un dispositif selon l'invention disposé parallèlement sur un fémur 2 fracturé d'une cuisse 1, d'un patient accidenté. Le dispositif est placé le long de la cuisse 1 et à l'extérieur de celle-ci. Le fémur 2 est représenté après réduction de la fracture.

Le dispositif comporte au moins deux fiches 3 vissées dans l'os 2 de part et d'autre de la fracture et solidarisées avec un support rigide 4 par un mécanisme de liaison.

Les fiches 3 de type connu, réalisées en métal inoxydable, sont de préférence munies d'une extrémité autoforante et peuvent avoir de 3 à 6mm de diamètre.

Le support rigide 4 est constitué d'un tube cylindrique lisse réalisé de manière connue par des enroulements de fibres de carbone.

Le mécanisme de liaison comprend l'association d'une prise 6 ou 7 de fiches avec un collier 5, à l'aide d'un moyen de fixation 8 unique.

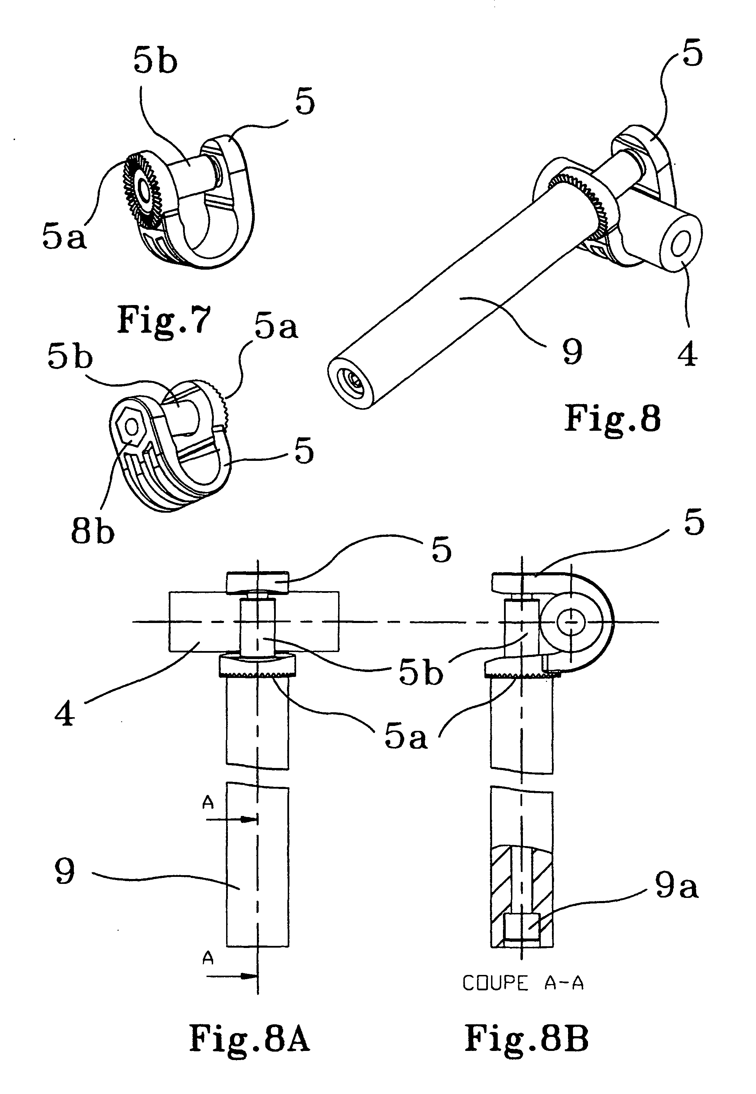

Les colliers 5, réalisés en un matériau synthétique souple, sont montés par clipsage sur le support rigide 4, et peuvent coulisser autour et le long de celui-ci. Ces colliers 5, adaptés au diamètre du support rigide et ayant la forme d'un U, sont perforés à chaque extrémité (figure 7). Un anneau crénelé 5a est pratiqué sur la partie externe de l'une des extrémités et une entretoise 5b amovible, constituée d'un tube cylindrique creux, est disposée entre les deux extrémités des colliers pour maintenir un écart constant entre elles. La partie externe de l'orifice percé dans cette extrémité présente une ouverture 5c de forme circulaire pour faciliter le positionnement relatif de chaque collier par rapport aux prises 6 ou 7 de fiches. Logé dans l'orifice de l'autre extrémité du collier, se trouve un insert 8b noyé dans l'épaisseur de celle-ci. Cet insert est muni d'un orifice fileté.

Le corps des deux types 6 et 7 de prises de fiches présente un perçage transversal.

Le moyen de fixation 8 est constitué d'une vis 8a filetée de type connu ayant une longueur suffisante pour traverser le corps d'une prise de fiche par le perçage transversal, l'entretoise 5b et les extrémités du collier correspondant. L'extrémité de la vis est engagée dans l'orifice fileté de l'insert 8b pour assurer, après serrage de la vis, le blocage dans toutes les directions du mécanisme de liaison et des fiches 3 correspondantes.

La longueur de l'entretoise 5b est adaptée pour limiter le couple de serrage de la vis 8a, tout en permettant une immobilisation complète du collier 5 par rapport au support rigide 4 après blocage de la vis 8a.

Le dispositif prévoit deux types différencs 6 et 7 de prises de fiches.

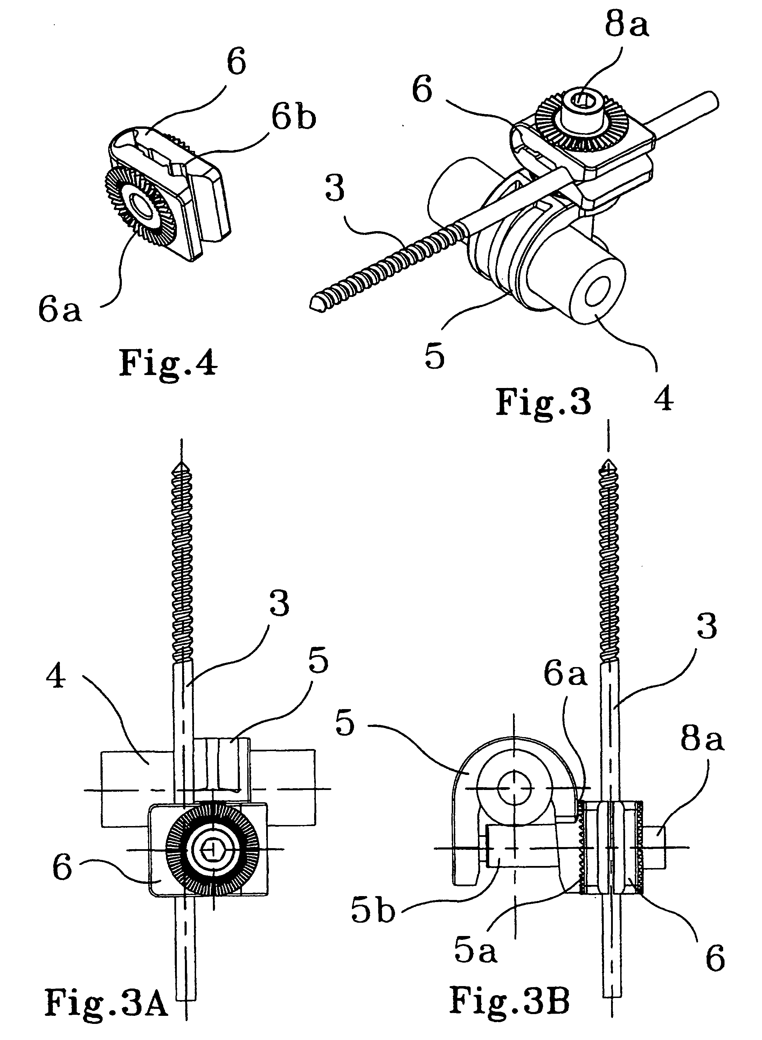

La prise 6 (figure 4) est constituée d'une pièce monobloc en forme de U comprenant deux éléments symétriques de forme rectangulaire, dont deux côtés sont reliés entre eux à l'aide d'un élément courbe et élastique, permettant d'insérer par clipsage l'extrémité d'au moins une fiche 3 entre les deux éléments rectangulaires. Les deux autres côtés de ces derniers comportent un biseau interne pour faciliter l'insertion de l'extrémité des fiches 3.

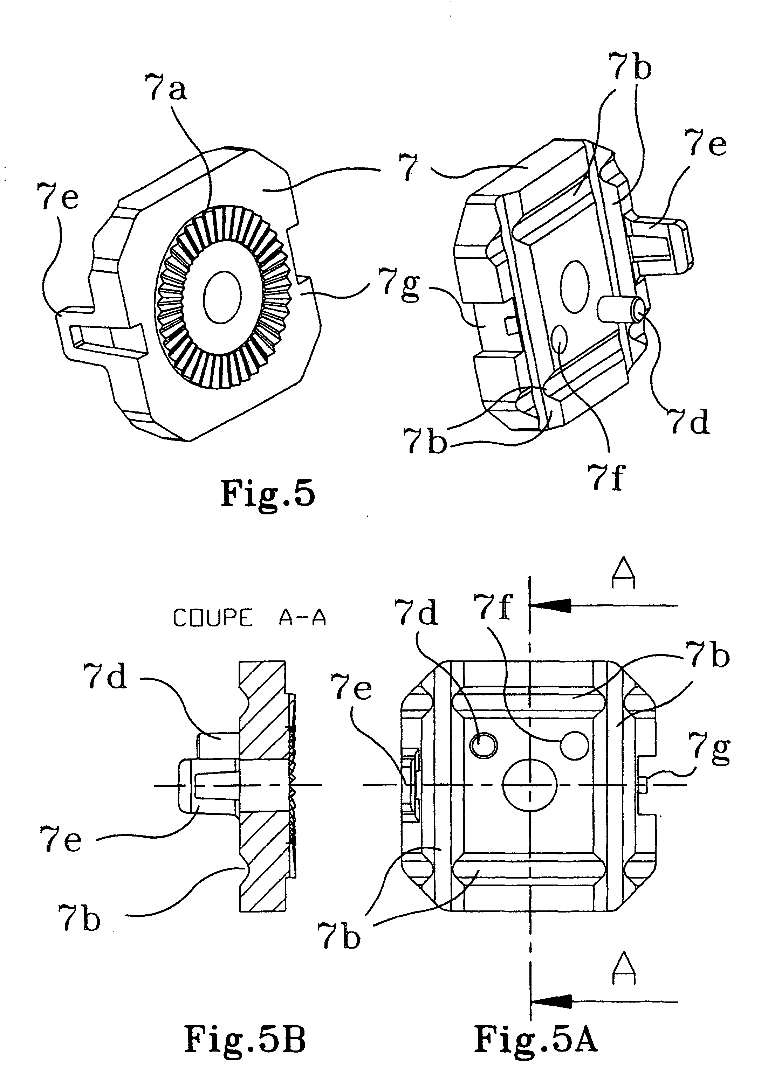

La prise 7 (figures 5 à 5B) en forme d'étau comprend deux pièces identiques dissociables de forme rectangulaire comportant un ergot 7d en saillie sur leur face interne, un orifice 7f transversal excentré, une encoche 7g pratiquée sur l'un des côtés et une languette 7e flexible fixée sur le côté opposé.

Lors de l'assemblage des deux pièces identiques, l'ergot 7d et la languette 7e de l'une des pièces sont logés respectivement dans l'orifice 7f et l'encoche 7g de l'autre pièce, pour former un étau dans lequel sont placées les extrémités de deux fiches 3. Si nécessaire, cette prise 7 est compatible pour recevoir l'extrémité d'une seule fiche 3.

Les faces externes des deux types 6 et 7 de prise de fiches présentent un anneau crénelé 6a et 7a identique à celui 5a des colliers 5. L'anneau crénelé 5a du collier est placé au contact de celui 6a ou 7a de la prise 6 ou 7 utilisée, pour assurer un indexage précis et optimiser leur cohésion et leur immobilisation par rapport au support rigide 4.

Dans l'épaisseur de la face interne des deux types 6 et 7 de prise de fiches sont pratiquées des empreintes 6b et 7b pour recevoir chacune l'extrémité d'une fiche 3. Ces empreintes 6b et 7b ont la forme d'une gorge de section triangulaire (figures 4 et 5B) sur la totalité de la longueur ou largeur de la prise 6 et 7 de fiches. La forme particulière de ces empreintes permet de supprimer toutes possibilités de rotation de l'extrémité cylindrique de la fiche à l'intérieur de la prise 6 ou 7.

Lors d'une fracture de l'os 2 au niveau de l'épiphyse 2a (figure 2), il est nécessaire de placer des fiches 3 dans cette épiphyse pour assurer une immobilisation correcte de l'os fracturé.

Pour cela, le dispositif comporte un support transversal 9 qui est solidarisé avec le support rigide 4 par un collier 5 décrit plus haut (figures 2 et 8 à 8B).

Ce support transversal 9 est constitué d'un petit tube cylindrique en carbone du même type que le support rigide 4. Il est percé suivant son axe longitudinal pour introduire par l'une de ses extrémités une vis 9a filetée (figure 8B), ou tout autre moyen équivalent, vissée sur l'insert 8b d'un collier 5, pour le solidariser avec le support rigide 4 après serrage de la vis 9a.

Cette vis est semblable à la vis 8a décrite plus haut, mais de longueur supérieure pour s'adapter à celle du support transversal 9 et à l'écartement des extrémités du collier 5.

Sur la section de l'autre extrémité du support transversal 9 est dessiné un anneau crénelé (non représenté) positionné sur celui 5a du collier 5, pour améliorer la fixation de ce support sur ce dernier en interdisant tout déplacement en rotation du support transversal 9. Sur ce support 9, de même diamètre que le support rigide 4, sont montés un ou plusieurs mécanismes de liaison tels que décrits ci-dessus.

A l'exception des fiches 3, la totalité du dispositif est réalisée en matériau synthétique radiotransparent et aamagnétique du type carbone.

Ce matériau très rigide est également très résistant à une température répétée de 140 °C et aux produits utilisés pour les opérations de stérilisation de l'ensemble du dispositif.

Pour faciliter le montage du dispositif sur le patient, il a été déterminé une couleur différente pour les différents types de pièces utilisées.

Le fonctionnement du dispositif orthopédique monolatéral de fixation externe est le suivant.

Après avoir réduit la fracture de l'os 2 et introduit au moins deux fiches 3 de part et d'autre de la fracture, le chirurgien procède au montage d'un mécanisme de liaison sur l'extrémité de chaque fiche.

Cette extrémité est engagée par clipsage dans la prise 6 monobloc et positionnée dans l'empreinte 6b. Un collier 5 est placé sur le support rigide 4 et une entretoise 5b est disposée entre les extrémités du collier 5.

A l'aide des éléments de positionnement 5c et 6c, le collier 5 et la prise 6 sont réunis en mettant en contact les anneaux crénelés 5a et 6a. Ainsi, les orifices de la prise et ceux du collier et de l'entretoise se trouvent alignés, pour introduire alors la vis 8a et procéder au blocage du mécanisme en serrant celle-ci à l'aide d'une clé, jusqu'à ce que l'entretoise 5b vienne en butée entre les extrémités du collier.

Pour solidariser deux fiches 3 voisines avec le support rigide 4, on place l'une des deux pièces constitutives d'une prise 7 en forme d'étau sur les extrémités des deux fiches, puis l'autre pièce est posée sur la précédente en faisant coïncider respectivement l'ergot 7d et la languette 7e de l'une avec l'orifice 7f et l'encoche 7g de l'autre. Simultanément, les extrémités des fiches 3 sont positionnées dans les empreintes 7b.

L'assemblage de cette prise 7 avec un collier 5 et le support rigide 4 reste identique à celui décrit ci-dessus.

Avant de serrer complètement la vis de fixation 8a, la disposition angulaire de chaque mécanisme de liaison par rapport aux fiches 3 et au support rigide 4 est réglée avec précision, en modifiant la position relative des anneaux crénelés 6a, 7a et 5a et en déplaçant en rotation et translation les colliers 5 sur le support rigide 4.

Pour le montage épiphysaire, un support transversal 9 est solidarisé avec le support rigide 4 à l'aide d'un collier 5, muni d'une entretoise 5b, placé au préalable sur le support rigide.

L'anneau crénelé (non représenté), dessiné sur la section de l'extrémité du support transversal 9, est positionné sur celui 5a du collier, puis la vis 9a est serrée jusqu'à mise en butée de l'entretoise 5b sur les extrémités du collier.

Le montage des mécanismes de liaison sur le support transversal 9 s'effectue de la même manière que celui décrit pour le support rigide 4.

De préférence, la vis 9a est maintenue prisonnière à l'intérieur du support transversal 9.

Ce dispositif comporte un nombre de pièces mises en oeuvre très faible avec une grande simplicité de montage, ce qui le rend totalement adapté aux interventions d'urgence réalisées dans des conditions particulièrement difficiles.

Bien entendu, diverses modifications peuvent être apportées par l'homme de l'art au dispositif orthopédique de fixation externe pour l'immobilisation d'un os fracturé qui vient d'être décrit, uniquement à titre d'exemple non limitatif, sans sortir du cadre de protection défini par les revendications annexées.

## Revendications

1. Dispositif orthopédique monolatéral de fixation externe pour l'immobilisation d'un os fracturé (2), comportant un support rigide (4), au moins deux fiches (3) aptes à être vissées dans l'os et solidarisées avec le support rigide par un mécanisme de liaison comprenant une prise (7) de fiches constitué de deux pièces identiques formant un étau, dont les deux faces en regard présentent des empreintes (7b) aptes à recevoir et à maintenir des fiches (3) ayant des diamètres de 3 à 6 mm, dispositif **caractérisé en ce que** les empreintes ont des directions différentes, les empreintes selon l'une des directions permettent le maintien des fiches de 3 ou 4 mm de diamètre parallèles entre elles et les empreintes selon l'autre direction, perpendiculaire à la précédente, permettent le maintien de fiches de 5 ou 6 mm de diamètre parallèles entre elles.

2. Dispositif selon la revendication 1, **caractérisé en ce que** le mécanisme de liaison comporte un collier (5) pouvant glisser autour du support (4) et une vis unique amovible assurant un blocage omnidirectionnel à la fois de chaque mécanisme de liaison et de chaque fiche (3) par rapport respectivement au support (4) et à sa prise (7) de fiches correspondante.

3. Dispositif selon l'une quelconque des revendications précédentes, **caractérisé en ce que** la prise de fiches comprend un mécanisme de détrompage guidant le positionnement d'une pièce formant étau par rapport à l'autre.

4. Dispositif selon la revendication 3, **caractérisé en ce que** le mécanisme de détrompage comprend sur chacune des pièces formant l'étau un ergot (7d) et une languette (7e) se logeant respectivement dans un orifice (7f) et une encoche (7g) de l'autre pièce identique.

5. Dispositif selon l'une quelconque des revendications précédentes, **caractérisé en ce que** deux surfaces crénelées (7a, 5a) sont réalisées respectivement sur chaque face en contact de la prise (7) de fiches et de chaque collier (5), pour assurer un indexage précis, une cohésion des pièces entre elles et une totale immobilisation des fiches par rapport au support rigide (4).

6. Dispositif selon l'une quelconque des revendications précédentes, **caractérisé en ce que** les colliers (5) comportent une entretoise (5b) pour maintenir un écart constant entre les deux extrémités du collier et limiter le couple de serrage de la vis (8a).

7. Dispositif selon l'une quelconque des revendications précédentes, caractérisé en ce le support rigide (4) est constitué d'un tube cylindrique réalisé en carbone.

8. Dispositif selon l'une quelconque des revendications précédentes, **caractérisé en ce que** la totalité du dispositif, excepté les fiches (3), est réalisée en matériau synthétique radiotransparent et amagnétique.

9. Dispositif selon l'une quelconque des revendications précédentes, **caractérisé en ce qu'**il comporte un support transversal (9), solidarisé au support rigide (4) par un collier (5), pour maintenir une épiphyse (2a) de l'os fracturé (2) à l'aide d'au moins une fiche (3) vissée dans l'épiphyse et d'un mécanisme de liaison (7, 5) monté sur le support transversal (9).

## Patentansprüche

1. Monolaterale orthopädische Vorrichtung zur externen Fixierung (Fixateur externe) zur Ruhigstellung eines gebrochenen Knochens (2), die einen starren Träger (4) enthält, mindestens zwei Stäbe (3), die sich dafür eignen, in einen Knochen eingedreht zu werden und die mit dem starren Träger verbunden sind durch einen Verbindungsmechanismus, der eine Stabaufnahme (7) aufweist, die aus zwei identischen Teilen besteht, die einen Schraubstock bilden, dessen zwei sich gegenüber liegenden Seiten Vertiefungen (7b) aufweisen, die dafür geeignet sind, Stäbe (3) mit einem Durchmesser von 3 mm bis 6 mm aufzunehmen und festzuhalten, wobei die Vorrichtung **dadurch gekennzeichnet ist, dass** die Vertiefungen verschiedene Richtungen aufweisen, wobei die Vertiefung entlang einer Richtung das Halten von parallel zueinander verlaufenden Stäben mit einem Durchmesser von 3 mm oder 4 mm erlauben und die Vertiefung entlang der anderen Richtung, die senkrecht zur vorhergehenden Richtung ist, das Halten von parallel zueinander stehenden Stäben mit einem Durchmesser von 5 mm oder 6 mm erlauben.

2. Vorrichtung nach Anspruch 1, **dadurch gekennzeichnet, dass** der Verbindungsmechanismus eine Schelle (5), die um den Träger (4) herum gleiten kann, und eine einzige abnehmbare Schraube umfasst, die eine Blockierung in allen Richtungen sowohl von jedem Verbindungsmechanismus als auch von jedem Stab (3) in bezug auf den Träger (4) und auf seine entsprechende Stabaufnahme (7) gewährleistet.

3. Vorrichtung nach einem der vorangehenden Ansprüche, **dadurch gekennzeichnet, dass** die Stabaufnahme einen Mechanismus zur Fehlervermeidung umfasst, der die Positionierung eines Teils, der einen Schraubstock bildet, in bezug auf das andere Teil steuert.

4. Vorrichtung nach Anspruch 3, **dadurch gekennzeichnet, dass** der Mechanismus zur Fehlervermeidung auf jedem Teil, das den Schraubstock bildet, einen Vorsprung (7d) und eine Lasche (7e) umfasst, die sich jeweils in eine Öffnung (7f) und in eine Einkerbung (7g) des anderen identischen Teils einfügen.

5. Vorrichtung nach einem der vorangehenden Ansprüche, **dadurch gekennzeichnet, dass** jeweils auf jeder Fläche, die mit der Stabaufnahme (7) und mit jeder Schelle (5) in Berührung ist, zwei gezahnte Oberflächen (7a, 5a) ausgebildet sind, um ein genaues Positionieren, einen Zusammenhalt zwischen den Teilen und eine vollkommene Ruhigstellung der Stäbe in bezug auf den starren Träger (4) zu gewährleisten.

6. Vorrichtung nach einem der vorangehenden Ansprüche, **dadurch gekennzeichnet, dass** die Schellen (5) eine Querstrebe (5b) aufweisen, um einen konstanten Abstand zwischen den zwei Enden der Schelle beizubehalten und um das Anziehdrehmoment der Schraube (8a) zu begrenzen.

7. Vorrichtung nach einem der vorangehenden Ansprüche, **dadurch gekennzeichnet, dass** der starre Träger (4) aus einem zylindrischen Rohr aus Karbon besteht.

8. vorrichtung nach einem der vorangehenden Ansprüche, **dadurch gekennzeichnet, dass** die gesamte Vorrichtung, ausgenommen die Stäbe (3), aus radiotransparentem und unmagnetischem synthetischem Material hergestellt ist.

9. Vorrichtung nach einem der vorangehenden Ansprüche, **dadurch gekennzeichnet, dass** sie einen transversalen Träger (9) umfasst, der mit einer Schelle (5) mit dem starren Träger (4) verbunden ist, um eine Epiphyse des gebrochenen Knochens (2) mit Hilfe mindestens eines Stabes (3) zu halten, der in die Epiphyse eingedreht ist, und eines Verbindungsmechanismus (7, 5), der auf dem transversalen Träger (9) montiert ist.

## Claims

1. A monolateral orthopaedic device with external fixing for immobilising a fractured bone (2), comprising a rigid support (4), at least two pins (3) capable of being screwed into the bone and joined to the rigid support by a connecting mechanism comprising a pin-holding device (7) consisting of two identical parts forming a clamp whose two opposing faces have depressions (7b) capable of receiving and holding pins (3) having diameters of from 3 to 6 mm, said device being **characterised in that** the depressions extend in different directions, the depressions in one of the directions allow pins 3 or 4 mm in diameter to be held parallel to one another and the depressions in the other direction, perpendicular to the previous direction, allow pins 5 or 6 mm in diameter to be held parallel to one another.

2. A device according to claim 1, **characterised in that** the connecting mechanism comprises a collar (5) which may slide around the support (4) and a single removable screw ensuring simultaneous omnidirectional locking of each connecting mechanism and each pin (3) relative respectively to the support (4) and its corresponding pin-holding device (7).

3. A device according to any one of the preceding claims, **characterised in that** the pin-holding device comprises a foolproofing mechanism guiding the positioning of one clamp-forming part relative to the other.

4. A device according to claim 3, **characterised in that** the foolproofing mechanism comprises on each of the clamp-forming parts a peg (7d) and a tongue (7e) accommodated respectively in an orifice (7f) and a notch (7g) of the other identical part.

5. A device according to any one of the preceding claims, **characterised in that** two serrated surfaces (7a, 5a) are formed respectively on each face in contact with the pin-holding device (7) and each collar (5), to ensure precise indexing, cohesion of the parts with each other and full immobilisation of the pins relative to the rigid support (4).

6. A device according to any one of the preceding claims, **characterised in that** the collars (5) comprise a spacer (5b) to maintain a constant distance between the two ends of the collar and to limit the tightening torque of the screw (8a).

7. A device according to any one of the preceding claims, **characterised in that** the rigid support (4) consists of a cylindrical tube made of carbon.

8. A device according to any one of the preceding claims, **characterised in that** the entire device, with the exception of the pins (3), is made of a synthetic radiotransparent, nonmagnetic material.

9. A device according to any one of the preceding claims, **characterised in that** it comprises a transverse support (9) joined to the rigid support (4) by a collar (5), to hold an epiphysis (2a) of the fractured bone (2) using at least one pin (3) screwed into the epiphysis and a connecting mechanism (7, 5) mounted on the transverse support (9).
